# EUROPEAN PATENT SPECIFICATION

(11) **EP 2 407 151 B1**
(45) Date of publication and mention of the grant of the patent: **02.10.2019**
(21) Application number: 10169695.3
(22) Date of filing: 15.07.2010
(51) Int. Cl.: A61K 9/06, A61Q 19/00, A61K 8/36, A61K 8/368, A61K 31/19, A61K 31/60, A61K 47/32, A61K 47/38

(54) **Composition for the treatment of skin and/or nail lesions**
Zusammensetzung zur Behandlung von Haut- und/oder Nagelverletzungen
Composition pour le traitement de lésions de la peau et/ou des ongles

(43) Date of publication of application: 18.01.2012
(73) Proprietor: Progressare Medinvest B.V., 1019 HC Amsterdam (NL)
(72) Inventor: Hendriks, Maikel, 1019 HC, Amsterdam (NL); Bouter, Pieternella Anna Maria, 1019 HC, Amsterdam (NL); Kolodziej, Katarzyna, 1019 HC, Amsterdam (NL)
(74) Representative: Ellens, Andries

(56) References cited:
- WO-A1-2010/105052
- WO-A2-01/05387
- WO-A2-2006/053223
- WO-A2-2006/077156
- US-A- 4 514 385
- US-A- 5 449 519
- US-A1- 2006 263 398
- DATABASE WPI Week 200577 Thomson Scientific, London, GB; AN 2005-752331 XP002391653, -& JP 2005 281133 A (SHISEIDO CO LTD) 13 October 2005 (2005-10-13)
- NGUYEN T H ET AL: "Trichloroacetic acid peels", DERMATOLOGIC THERAPY 2000 DK LNKD- DOI:10.1046/J.1529-8019.2000.00020.X, vol. 13, no. 2, 2000, pages 173-182, XP002614107, ISSN: 1396-0296
- ALLEN LLOYD, V JR: "Basics of Compounding for the Treatment of Warts", INTERNATIONAL JOURNAL OF PHARMACEUTICAL COMPOUNDING, March 2004 (2004-03), - April 2004 (2004-04), pages 126-129,

## Description

### FIELD OF THE INVENTION

The invention relates to a composition for the treatment of skin lesions and/or nail lesions, an applicator comprising such a composition and the use of such a composition.

### BACKGROUND OF THE INVENTION

Various compositions are known for the treatment of skin lesions such as warts, corn and calluses, and/or nail lesions such as ingrown toenails. Many known compositions are ineffective

### OBJECT AND SUMMARY OF THE INVENTION

It is an object of the invention to provide an effective and improved composition for the treatment of skin lesions and/or nail lesions.

The invention provides a composition for the treatment of skin lesions and/or nail lesions, comprising an effective amount of trichloroacetic acid, at least one thickener (for increasing the viscosity of the solvent), and at least one physiologically acceptable liquid carrier, wherein the composition comprises at least 20% w/w trichloroacetic acid, and 0.5-3% w/w carbomer. Trichloroacetic acid (TCA) in the composition according to the invention, proved to be effective against a plethora of skin lesions, in particular warts, corn and calluses, as well as nail lesions including ingrown toenails and onychomycosis. Salicylic acid is another component effective in corroding the skin or nails, and may be used in combination with trichloroacetic acid. Application of Trichloroacetic was particularly effective against genital warts, and especially against anogenital warts where it has been studied in comparative trials where its effectiveness was compared with other anti-wart therapies. In addition, TCA application was effective various other skin disorders, in particular: epidermodysplasia veruciformis- skin disorder also caused by HPV virus, acne, to remove acne scars and wrinkles, tattoo removal, eyelid xanthelasma, and solar lentigo, melasma.

Salicylic acid has a positive effect in treating skin and nails. In addition to that, salicylic acid gives a mild anaesthetic effect. Preferably, salicylic acid is used in concentrations ranging from 1%-20% w/w.

Salicylic acid in combination with TCA showed a synergistic effect against skin lesions and nail lesions. Another advantage is that using a combination of salicylic acid and TCA allows for a composition with a relatively a low concentration of each of salicylic acid and TCA with a similar effect to compositions using only TCA or only salicylic acid. Using the relatively low concentrations of TCA and salicylic acid decreases the chance of skin irritation due to either of these compounds. Moreover, salicylic acid diminished the discomforting burning feeling on skin and nails sometimes experienced by persons treated with products containing substantial amounts of TCA.

The composition according to the invention may comprise other ingredients commonly used in cosmetics and pharmaceutical products, such as surfactants, emulsifiers, colorants and perfumes.

An effective amount of TCA of preferably at least 0.1% by weight, more preferably at least 1% w/w is disclosed. For lower concentrations, the composition is too low to work efficiently. Compositions comprising TCA preferably in the range of 0.1-50% w/w are also disclosed. Compositions according to the invention must contain at least 20% w/w TCA. Concentrations higher than 50% TCA w/w are also effective, but have an increased risk for complications and should therefore only be applied by skilled persons.

The composition is preferably a fluid, in order to enable easy application and dividing the active ingredient on a skin or nail surface. The composition can be used in for instance a skin peeling treatment, for the medical or cosmetic treatment of skin lesion selected from the group consisting of warts, corn and calluses, and for nail treatments including ingrown toenails. A postulated mechanism of action is that the composition comprising TCA softens the skin or nail, and enables to peel the skin or nail lesion away. For severe lesions, multiple treatments may be needed. Both cosmetic and medical treatments may be performed with the compositions according to the invention.

The liquid carrier may be a single solvent or mixture of solvents and additives capable of dissolving of mixing with the concentration of TCA used. A preferred liquid carrier is water or a water-based mixture. Water may be mixed with an organic solvent. It is also possible to use a water-free carrier, preferably using easily evaporable solvents. The use of evaporable solvents makes it possible to achieve a relatively high concentration of TCA at a treated location on the skin or nail. Suitable evaporable solvents include methanol, ethanol, propanol, methyl ethyl ketone, acetone, ethyl acetate, and mixtures thereof.

The thickener provides the composition with an increased viscosity, making it easier to focus the active ingredient on the intended location on the skin or nails, preventing to some extend the spreading of the composition to locations where its action is not desired. Also, the thickener improves the time the active ingredient remains on a treated surface, improving the efficacy of the composition. The thickener may be a single compound, but may also comprise a mixture of compounds. The thickener is preferably a gel-forming agent compatible with the liquid carrier used.

Preferably, the composition has a viscosity of at least 3 mPas as measured using rotary viscometry at 25°C. Viscosity is measured according to the rotating viscosimeter protocol in the European Pharmacopeia Ph.Eur (01/2005:20210) Ph. Eur. 5th edition vol 1, p. 29, chapter 2.2.10. Such compositions have a significant adhering effect on skin and/or nail, allowing for spot treatment. Compositions with viscosities up to about 60.000 mPas are considered to be useful; liquid compositions with higher viscosities are considered to be difficult to handle

In a preferred embodiment, the composition is a sprayable liquid having a viscosity in the range of about 5 mPas to about 2000 mPas at 25°C. Spraying is an easy and fast way to apply the composition to a surface to be treated. In another preferred embodiment, the composition has a viscosity of at least 5000 mPas as measured at 25°C according to the rotating viscosimeter protocol in the European Pharmacopeia Ph.Eur (01/2005:20210) Ph. Eur. 5th edition vol 1, p. 29, chapter 2.2.10. Rotating Viscometer Method . Such a viscosity gives the composition a particularly useful adhering effect on skin and nails. Preferably, the composition is a gel composition having a viscosity in the range of about 3000 mPas to 60000 mPas at 25°C. The gel composition is relatively easy to apply, either manually or by using a suitable applicator, and combines a relatively large adhering effect. The gel is particularly suitable to apply the composition selectively to a specific spot, such as a wart.

Preferably, the composition has a viscosity in the range of 5000 mPas to 10000 mPas at 25°C. Such a composition shows a sufficient adhering effect, while still being relatively easy to apply and process. It is advantageous if the liquid carrier is an aqueous carrier. A water-based composition is relatively easy to prepare, and dissolves TCA well. The water may be mixed with other solvents, for instance C1-C6 alcohols or ketones, and additives such as surfactants. Preferably, the liquid carrier consists for at least 50% of water. Preferably, the composition has a pH below 4. Low pH compositions appear to provide a better treatment results. In addition, the low pH compositions appear to have an additional effect in suppressing microbiological threats on the skin and nail, including bacteria, fungus and yeast, in particular onchyomycosis (nail fungus).

The composition comprises at least 20% w/w trichloroacetic acid, preferably in the range of 20-50% w/w. Compositions having a concentration of at least 20% w/w show good results in treating skin and nail lesions within a relatively short treatment. Although skin irritations occasionally occur after treatment with TCA concentrations below 50%, TCA concentrations over 50% were found to significantly increase the chance of skin irritations.

It is preferred if the thickener comprises at least one polysaccharide thickener. Polysaccharide thickeners showed in addition to their thickening effect to decrease the chance of skin irritations.

In a preferred disclosure, the thickener comprises at least one thickener selected from the group consisting of amylose, amylopectine, carbopol, cellulose, carboxymethyl cellulose or salts thereof, ethyl cellulose, hydroxypropyl cellulose and methyl cellulose. The viscosity of the formulation having excellent adhering effects may be achieved using these thickeners.

Compositions according to the invention comprise a carbomer. Carbomer includes the commercially available products Carbopol 71 G NF,Carbopol 971P NF, Carbopol 974P NF, Carbopol 934P NF, Carbopol 980P NF, Carbopol 981P NF, Carbopol 5984EP, Carbopol ETD 2020 NF, Carbopol 934 NF, Carbopol 934P NF, Carbopol 940 NF, Carbopol 941 NF, Carbopol 1342 NF, Pemulen TR-1 NF, Pemulen TR2-NF, Noveon AA-USP, and Carbopol Ultrez 10 NF.

Preferably, the thickener remains essentially stable in the presence of trichloroacetic acid. Some thickeners are degraded in the presence of TCA, resulting in a coloured product, which may appear less attractive to some persons. Also, the color of a solution may be difficult to control in case a colorant was used. A colourless composition is generally considered to be visually more attractive and therefore more desirable.

The thickener comprises carbopol. Carbopol shows an excellent stability in the presence of TCA, allowing for colorless compositions, or excellent color control in case the composition comprises a colorant. Carbopol polymers, also called carbomers, are polymers of acrylic acid cross-linked with polyalkenyl ethers or divinyl glycol.

Carbopol allows for an excellent control of the viscosity of the formulation.

The composition comprises carbopol in the range of 0.5-3% w/w. Compositions comprising 0.5%-1% w/w carbopol are excellent for sprayable products, products comprising 1.5-2.5% w/w form a well-applicable gel. Products comprising carbopol more than 3% w/w may become too rigid for easy processing and application.

In a preferred embodiment, the composition comprises a combination of carbopol and glycerine. Although glycerine is by itself not a thickener, the addition of glycerine to a composition according to the invention using carbopol yields an increase in viscosity. Thus the cost price for a formulation with a relatively high viscosity may be reduced by using less of the relatively expensive carbopol by adding the relatively cheap glycerine. Preferably, glycerine is added in an amount ranging from approximately 1% w/w to approximately 20% w/w. As an additional effect, glycerine was found to reduce the chance of skin irritation by TCA.

The invention also provides an applicator device comprising a composition according to any of the preceding claims. The applicator may be design for efficient application of the composition depending on its intended purpose (for instance skin of nail treatment), and considering the viscosity of the formulation. The applicator device may for instance be a pen applicator, a brush applicator, roll-on applicator or a spray applicator.

The invention further provides the use of a composition according to the invention for the cosmetic treatment of the human skin.

Preferably, the composition is used for a skin peeling treatment or the treatment of a skin lesion selected from the group consisting of warts, corn and calluses. These treatments are typically considered cosmetic treatments.

The compositions can also be used for the cosmetic treatment of nails. In particular nail deformations (for instance as a result from onychomycosis) and ingrown toenails may be treated effectively.

Consequently, the invention also provides the use of a composition according to any of claims 1-12 for the non-therapeutic, cosmetic treatment of calluses and corns of the human skin, or of ingrown human toenails.

### DESCRIPTION OF PREFERRED EMBODIMENTS

The invention will now be further elucidated by the following non-limiting examples.

### Methods

TCA is the abbreviation for trichloroacetic acid. Ingredients were mixed using conventional mixing techniques. pH of the compositions was determined at room temperature (25 °C) using a commercially available digital pH meter and/or universal pH indicator test strips.

Viscosity was measured in a commercially available Brookfield RV rotational viscosity meter at room temperature (25 °C), at 20 rpm, according to the method described in Ph. Eur. 5th edition vol 1, p. 29, chapter 2.2.10. Rotating Viscometer Method.

### Examples

The next tables show various compositions prepared by mixing the appropriate ingredients using regular methods. Spray 2 is not according to the invention.

**Example 1 : spray compositions**

| Ingredient % (w/w) | Spray 1 | Spray 2 | Spray 3 |
|---|---|---|---|
| TCA | 20 | - | 20 |
| Salicylic acid | - | 2 | 2 |
| carbopol | 0.5 | 0.5 | 0.5 |
| water | Fill to100% | Fill to100% | Fill to100% |
| pH | 2 | 3 | 3 |
| Viscosity (mPas) | 5 | 5 | 5 |

These compositions have a relatively low viscosity and are therefore fast and easy to apply by a spray applicator, either from a canister using a propellant gas, or a manual pump system. The low viscosity makes it easy to treat a relatively large skin or nail area. The carbopol thickener ensures that the liquid composition adheres to the treated area.

These compositions are particularly suitable for treating calluses and for the whole body in light and medium-deep chemical peel treatments. In the treatment of corns or calluses, or other thickened skin lesions, the composition is sprayed on the lesions and allowed to work for at least 1 hour, preferably 3-8 hours. Afterwards, remainders of the composition may be washed off using water.

For chemical peels, the composition is applied over a treated skin area (preferably by spraying) and allowed to react for 5-30 minutes, after which the composition may be washed off using water. Optionally, a neutralizing pH buffer or other neutralizing agent may be used in case a burning sensation and skin irritation occurs, in particular for the compositions comprising TCA.

Spray 1 (TCA) showed better and faster results than spray 2 (salicylic acid). Spray 3, using a combination of TCA and salicylic acid, showed a better and faster effect than either spray 1 or spray 2, and users experienced a diminished burning feeling and lower chance of skin irritation compared to the formulations comprising TCA without salicylic acid.

**Example 2: hard gel compositions:**

| Ingredient % (w/w) | Gel 1 | Gel 2 | Gel 3 |
|---|---|---|---|
| TCA | 40 | - | 40 |
| Salicylic acid | - | 2 | 2 |
| carbopol | 2 | 2 | 2 |
| glycerine | 12 | 12 | 12 |
| water | Fill to100% | Fill to100% | Fill to100% |
| pH | 2 | 3 | 2 |
| viscosity | 8000 | 8000 | 8000 |

Gel 2 is not according to the invention. The gel compositions are easily applied locally, either manually or by an applicator. The carbopol thickener ensures that the composition has a sufficient viscosity to retain the active ingredient at the selected area to be treated (for instance a toe nail), while diminishing spreading of the active ingredient over to areas that do not need treatment. This lowers the chance of unnecessary skin irritation outside the area or spot that needs treatment.

Varying the carboxymethylcellulose amount from 0.5-2% w/w yields compositions with a viscosity ranging from 5000-10000 MPas.

It was discovered that adding glycerine enhances the thickening effect of carbopol and lowers the chance for skin irritation.

These gel compositions are particularly suitable for local treatment of lesions, in particular warts, corns, calluses and ingrown toenails. The compositions are applied to the lesions and allowed to work for at least 10 minutes, preferably a number of hours, and will not necessarily have to be washed off, unless skin irritations occur.

Gel 1 (TCA) showed better and faster results than gel 2 (salicylic acid). Gel 3, using a combination of TCA and salicylic acid, showed a better and faster effect than either gel 1 or gel 2, and users experienced a diminished burning feeling and lower chance of skin irritation compared to the formulations comprising TCA without salicylic acid .

**Reference example 3: gel composition:**

| Ingredient % (w/w) | Gel 4 | Gel 5 | Gel 6 |
|---|---|---|---|
| TCA | 40 | - | 40 |
| Salicylic acid | - | 2 | 2 |
| carboxymethylcellulose | 4 | 4 | 4 |
| water | Fill to100% | Fill to100% | Fill to100% |
| pH | 2 | 3 | 2 |
| viscosity | 8000 | 8000 | 8000 |

The gel composition is easily applied locally, either manually or by an applicator. The carboxymethylcellulose thickener ensures that the composition has a sufficient viscosity to retain the active ingredient at the selected area to be treated (for instance a toe nail), while diminishing spreading of the active ingredient over to areas that do not need treatment. Carboxymethylcellulose and other cellulose derivatives are particularly suitable as thickeners for TCA compositions, as cellulose derivatives were found to lower the chance of skin irritation by TCA. The amount of carboxymethylcellulose or an equivalent cellulose derivative may be varied depending on the desired viscosity. Varying the carboxymethylcellulose amount from 2-5% w/w yields compositions with a viscosity ranging from 5000-10000 MPas.

These compositions are particularly suitable for treating warts, corns and calluses. The composition are applied to the lesions and allowed to work for at least 10 minutes, preferably at least a 1 hour.

Gel 4 (TCA) showed better and faster results than gel 5 (salicylic acid). Gel 6, using a combination of TCA and salicylic acid, showed a better and faster effect than either gel 4 or gel 5, and users experienced a diminished burning feeling and lower chance of skin irritation compared to the formulations comprising TCA without salicylic acid .

**Reference example 4: water-free gel composition:**

| Ingredient % (w/w) | Gel 7 | Gel 8 | Gel 9 |
|---|---|---|---|
| TCA | 30 | - | 30 |
| Salicylic acid | - | 2 | 2 |
| hydroxypropylcellulose | 2 | 2 | 2 |
| Castor oil | 4 | 4 | 4 |
| Methanol | 20 | 20 | 20 |
| Acetone | Fill to100% | Fill to100% | Fill to100% |
| pH* | 2 | 3 | 2 |
| viscosity | 3000 | 3000 | 3000 |

| | | | |
|---|---|---|---|
| *as determined by using water-wetted multipurpose pH test strips. | | | |

The gel composition is easily applied locally, either manually or by an applicator. The hydroxypropylcellulose thickener ensures that the composition has a sufficient viscosity to retain the active ingredient at the selected area to be treated (for instance a toe nail), while diminishing spreading of the active ingredient over to areas that do not need treatment. hydroxypropylcellulose and other cellulose derivatives are particularly suitable as thickeners for TCA compositions. The use of evaporable solvents makes it possible to achieve a relatively high concentration at a treated location on the skin or nail. Suitable evaporable solvents include methanol, ethanol, propanol, methyl ethyl ketone, acetone, ethyl acetate, and mixtures thereof.

These compositions are particularly suitable for treating warts, calluses, corns and ingrown nail. The compositions are applied to the lesions and allowed to work for at least 10 minutes, preferably at least 1 hour.

Gel 7 (TCA) showed better and faster results than gel 8 (salicylic acid). Gel 9, using a combination of TCA and salicylic acid, showed a better and faster effect than either gel 7 or gel 8, and users experienced a diminished burning feeling and lower chance of skin irritation compared to the formulations comprising TCA without salicylic acid .

## Claims

1. Composition for the treatment of skin lesions and/or nail lesions, comprising
- an effective amount of trichloroacetic acid,
- at least one thickener (for increasing the viscosity of the solvent), and
- at least one physiologically acceptable liquid carrier,
wherein the composition comprises at least 20% w/w trichloroacetic acid, and 0.5-3% w/w carbomer.

2. Composition according to claim 1, wherein the composition has a viscosity of at least 3 mPas as measured using rotary viscometry at 25°C.

3. Composition according to claim 2, wherein the composition has a viscosity of at least 5000 mPas at 25°C.

4. Composition according to any of claims 2 or 3, wherein the composition has a viscosity in the range of 5000 mPas to 10000 mPas at 25°C.

5. Composition according to any of the preceding claims, wherein the liquid carrier is an aqueous carrier.

6. Composition according to any of the preceding claims, wherein the composition has a pH below 4.

7. Composition according to any of the preceding claims, wherein the composition comprises trichloroacetic acid in the range of 20-50% w/w.

8. Composition according to any of the preceding claims, wherein the composition comprises a combination of trichloroacetic acid and salicylic acid.

9. Composition according to any of the preceding claims, wherein the thickener comprises at least one polysaccharide thickener.

10. Composition according to any of the preceding claims, wherein the composition forms a sprayable product comprising 0.5-1% w/w carbomer.

11. Composition according to any of the preceding claims, wherein the composition forms an application gel comprising 1.5-2.5% w/w carbomer.

12. Composition according to any of the preceding claims, wherein the composition comprises a combination of carbomer and glycerin.

13. Applicator device comprising a composition according to any of the preceding claims.

14. Use of a composition according to any of the claims 1-12 for the non-therapeutic, cosmetic treatment of calluses and corns of human skin.

15. Use of a composition according to any of the claims 1-12 for the non-therapeutic, cosmetic treatment of ingrown human toenails.

## Patentansprüche

1. Zusammensetzung zur Behandlung von Haut- und/oder Nagelverletzungen, umfassend
- eine wirksame Menge an Trichloressigsäure,
- mindestens ein Verdickungsmittel (zur Erhöhung der Viskosität des Lösungsmittels) und
- mindestens einen physiologisch verträglichen flüssigen Träger,
wobei die Zusammensetzung mindestens 20% Gew./Gew. Trichloressigsäure und 0,5-3% Gew./Gew. Carbomer umfasst.

2. Zusammensetzung nach Anspruch 1, wobei die Zusammensetzung eine Viskosität von mindestens 3 mPas, gemessen mittels Rotationsviskosimetrie bei 25°C, aufweist.

3. Zusammensetzung nach Anspruch 2, wobei die Zusammensetzung eine Viskosität von mindestens 5000 mPas bei 25°C aufweist.

4. Zusammensetzung nach einem der Ansprüche 2 oder 3, wobei die Zusammensetzung eine Viskosität im Bereich von 5000 mPas bis 10000 mPas bei 25°C aufweist.

5. Zusammensetzung nach einem der vorstehenden Ansprüche, wobei der flüssige Träger ein wässriger Träger ist.

6. Zusammensetzung nach einem der vorstehenden Ansprüche, wobei die Zusammensetzung einen pH-Wert unter 4 aufweist.

7. Zusammensetzung nach einem der vorstehenden Ansprüche, wobei die Zusammensetzung Trichloressigsäure im Bereich von 20-50% Gew./Gew. umfasst.

8. Zusammensetzung nach einem der vorstehenden Ansprüche, wobei die Zusammensetzung eine Kombination aus Trichloressigsäure und Salicylsäure umfasst.

9. Zusammensetzung nach einem der vorstehenden Ansprüche, wobei das Verdickungsmittel mindestens ein Polysaccharid-Verdickungsmittel umfasst.

10. Zusammensetzung nach einem der vorstehenden Ansprüche, wobei die Zusammensetzung ein sprühbares Produkt bildet, das 0,5-1% Gew./Gew. Carbomer umfasst.

11. Zusammensetzung nach einem der vorstehenden Ansprüche, wobei die Zusammensetzung ein Applikationsgel bildet, das 1,5-2,5 % Gew./Gew. Carbomer umfasst.

12. Zusammensetzung nach einem der vorstehenden Ansprüche, wobei die Zusammensetzung eine Kombination aus Carbomer und Glycerin umfasst.

13. Applikatorvorrichtung, umfassend eine Zusammensetzung nach einem der vorstehenden Ansprüche.

14. Verwendung einer Zusammensetzung nach einem der Ansprüche 1-12 für die nicht therapeutische, kosmetische Behandlung von Schwielen und Hühneraugen der menschlichen Haut.

15. Verwendung einer Zusammensetzung nach einem der Ansprüche 1-12 zur nicht therapeutischen, kosmetischen Behandlung von eingewachsenen menschlichen Zehennägeln.

## Revendications

1. Composition pour le traitement de lésions cutanées et/ou de lésions d'ongles, comprenant
- une quantité efficace d'acide trichloroacétique,
- au moins un épaississant (pour augmenter la viscosité du solvant), et
- au moins un véhicule liquide physiologiquement acceptable,
où la composition comprend au moins 20% p/p d'acide trichloroacétique et 0,5 à 3 % p/p de carbomère.

2. Composition selon la revendication 1, dans laquelle la composition a une viscosité d'au moins 3 mPa.s telle que mesurée par viscosimétrie rotative à 25 °C.

3. Composition selon la revendication 2, dans laquelle la composition a une viscosité d'au moins 5 000 mPa.s à 25 °C.

4. Composition selon l'une quelconque des revendications 2 ou 3, dans laquelle la composition a une viscosité dans l'intervalle de 5 000 mPa.s à 10 000 mPa.s à 25 °C.

5. Composition selon l'une quelconque des revendications précédentes, dans laquelle le véhicule liquide est un véhicule aqueux.

6. Composition selon l'une quelconque des revendications précédentes, dans laquelle la composition a un pH inférieur à 4.

7. Composition selon l'une quelconque des revendications précédentes, dans laquelle la composition comprend de l'acide trichloroacétique dans l'intervalle de 20 à 50% p/p.

8. Composition selon l'une quelconque des revendications précédentes, dans laquelle la composition comprend une combinaison d'acide trichloroacétique et d'acide salicylique.

9. Composition selon l'une quelconque des revendications précédentes, dans laquelle l'épaississant comprend au moins un épaississant polysaccharide.

10. Composition selon l'une quelconque des revendications précédentes, dans laquelle la composition forme un produit pulvérisable comprenant 0,5 à 1 % p/p de carbomère.

11. Composition selon l'une quelconque des revendications précédentes, dans laquelle la composition forme un gel d'application comprenant 1,5 à 2,5 % p/p de carbomère.

12. Composition selon l'une quelconque des revendications précédentes, dans laquelle la composition comprend une combinaison de carbomère et de glycérine.

13. Dispositif applicateur comprenant une composition selon l'une quelconque des revendications précédentes.

14. Utilisation d'une composition selon l'une quelconque des revendications 1 à 12 pour le traitement cosmétique non-thérapeutique des cals et des cors de la peau humaine.

15. Utilisation d'une composition selon l'une quelconque des revendications 1 à 12 pour le traitement cosmétique non-thérapeutique d'ongles humains incarnés.
